(19)

**Europäisches Patentamt**
**European Patent Office**
**Office européen des brevets**

(11) **EP 4 667 947 A1**

(12) **EUROPEAN PATENT APPLICATION**
published in accordance with Art. 153(4) EPC

(43) Date of publication:
**24.12.2025 Bulletin 2025/52**

(21) Application number: **24756138.4**

(22) Date of filing: **05.02.2024**

(51) International Patent Classification (IPC):
***G01R 19/00*** (2006.01)   ***A61B 17/22*** (2006.01)

(52) Cooperative Patent Classification (CPC):
**A61B 17/22; G01R 19/00;** Y02T 10/70

(86) International application number:
**PCT/CN2024/076190**

(87) International publication number:
**WO 2024/169764 (22.08.2024 Gazette 2024/34)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **16.02.2023  CN 202310126146**

(71) Applicant: **Jiangsu Polylive Medtech Co., Ltd.**
**Nantong, Jiangsu 226400 (CN)**

(72) Inventors:
• **TANG, Zhihuang**
  **Nantong, Jiangsu 226400 (CN)**
• **LI, Chuang**
  **Nantong, Jiangsu 226400 (CN)**
• **ZHOU, Yi**
  **Nantong, Jiangsu 226400 (CN)**
• **GUI, Baozhu**
  **Nantong, Jiangsu 226400 (CN)**

(74) Representative: **Metida**
**Gyneju str. 16**
**01109 Vilnius (LT)**

(54) **CURRENT DETECTION DEVICE, CONTROL METHOD, AND VASCULAR CALCIFICATION TREATMENT APPARATUS**

(57)    Disclosed in the present invention are a current detection device, a control method, and a vascular calcification treatment apparatus. The current detection device comprises a current detection module (110), a processing module (130), and a control module (140); the current detection module (110) is arranged in a high-voltage discharge circuit and is used for performing current detection on a first branch and a second branch of the high-voltage discharge circuit so as to respectively obtain a first current signal (I2) and a second current signal (I3); the processing module (130) is coupled to the current detection module (110); the control module (140) is connected to the processing module (130) and the current detection module (110) and is used for sending a pulse signal when receiving a discharge anomaly prompt signal sent by the processing module (130) so as to disconnect the first branch and the second branch. The present invention can be used for avoiding serious current unbalance of the first branch and the second branch which are connected in parallel in the high-voltage discharge circuit, thereby improving the stability of a system and the safety of an apparatus.

FIG. 1

EP 4 667 947 A1

## Description

This application claims the benefit of Chinese Patent Application No. 202310126146.X, filed on 16 February 2023, entitled "CURRENT DETECTION DEVICE, CONTROL METHOD, AND VASCULAR CALCIFICATION TREATMENT APPARATUS" , the entire contents of which are incorporated herein by reference.

Technical Field

The present invention relates to the technical field of electronic circuits, and in particular to a current detection device, a control method, and a vascular calcification treatment apparatus.

Description of Related Art

Vascular calcification is a common pathological manifestation universally present in atherosclerosis, hypertension, diabetic vascular lesions, vascular injury, chronic kidney disease, and aging. Its main manifestations include increased stiffness of the vascular wall and decreased compliance, which can easily lead to myocardial ischemia, left ventricular hypertrophy, and heart failure, trigger thrombosis and plaque rupture, and is one of the important factors for high incidence and mortality of cardiovascular and cerebrovascular diseases; it is also an important marker for atherosclerotic cardiovascular events, stroke, and peripheral vascular disease.

For vascular calcification lesions, the currently commonly used clinical treatment methods include non-compliant balloons, cutting balloons, plaque rotational atherectomy, and excimer lasers. However, these treatment methods are only suitable for mild to moderate calcification lesions and are difficult to treat deep calcification lesions. As an emerging technology, lithotripsy combines traditional electrohydraulic lithotripsy and balloon angioplasty, and can efficiently and safely pre-treat moderate to severe calcification lesions in coronary or peripheral arteries. It fractures calcified plaques without damaging the vascular endothelium, and can achieve good therapeutic effects on calcified nodules, eccentric calcification, superficial and deep calcification lesions.

The shock wave energy system based on electrohydraulic lithotripsy and balloon angioplasty consists of a shock wave generator, a connector, and a shock wave catheter. The shock wave generator is used to generate high-voltage pulse signals required for treatment, the connector connects the shock wave generator and the shock wave catheter to transmit pulse signals, after the high-voltage pulse signal reaches the shock wave catheter, it acts on the conductive medium inside the catheter balloon, the conductive medium breaks down to generate shock waves, and under the action of shock wave pressure and the tension of the vascular calcification itself, the vascular calcification is broken. The principle of the shock wave generator is mainly to use high voltage, large current, and instantaneous discharge. During the entire process, a high-energy-density high-voltage and high-temperature plasma zone appears on the discharge channel, converting electrical energy into acoustic energy, light energy, mechanical energy, and thermal energy. At the same time, the entire discharge channel will expand rapidly and form a pressure pulse in the liquid medium to form a shock wave.

High-voltage energy is released rapidly in a short time. How to accurately and quickly control shock wave energy is critical. To obtain greater pulse power, the system must have very high voltage or provide large pulse current, so the circuit needs appropriate high-voltage or large-current DC switches to complete high-voltage isolation and large-current path switching functions. The IGBT (Insulated Gate Bipolar Transistor) switch element is a fully controlled field-effect transistor device with very small turn-on and turn-off losses and has mature applications in various aspects, with switching action times in the microsecond or even nanosecond range. It is the best choice for DC large-current switch applications with strict requirements on turn-on and turn-off times. However, due to the influence of the semiconductor power devices, process materials, and other inherent performance of the IGBT module, the output current of a single IGBT cannot meet the current requirements of the shock wave equipment. At the moment of turn-on and turn-off, the IGBT will withstand extremely large surge current and peak voltage, which will inevitably increase IGBT power consumption, cause module overheating, and in severe cases, cause device failure or even damage to the main circuit. In practical applications, to carry higher output current, IGBTs are usually used in parallel, which can not only provide the required current but also form a redundant structure to improve system stability.

However, when IGBTs are operated in parallel, due to the dispersion of parameters of each module, the output current of each module cannot be exactly the same, resulting in some modules being overloaded and others being underloaded. This will reduce system stability and may cause serious consequences for system operation, and also greatly shorten the life of the IGBT module itself. Therefore, when used in parallel, the current of each IGBT branch needs to be considered. In shock wave treatment equipment, the high-voltage discharge time is short, the discharge current is large, and the impact on the IGBT is extremely large. If the current of the IGBT branch is uneven, it will accelerate the aging of the IGBT module in mild cases, greatly shortening the life of the IGBT and the shock wave equipment. In severe cases, serious uneven current in the IGBT branch will cause the IGBT module to fail and be damaged, resulting in abnormal equipment discharge and equipment damage.

## BRIEF SUMMARY OF THE INVENTION

Technical Problem

The shock wave equipment for intravascular calcification treatment is based on the principles of electrohydraulic effect and balloon angioplasty. High-voltage energy is rapidly released in the liquid channel, and extremely large currents are generated in the high-voltage discharge circuit. Due to the outstanding performance of IGBTs in turn-on and turn-off times and DC large-current applications, IGBTs are generally used for circuit control, but a single IGBT is difficult to carry the large current generated during discharge. Therefore, IGBTs are generally used in parallel to improve the ability to withstand load current. However, during parallel use, due to the dispersion of parameters of each module, the output current of each module cannot be exactly the same, some IGBT modules are overloaded, others are underloaded, which reduces system stability and also greatly shortens the life of the IGBT itself. The current shock wave equipment generally does not perform current sharing detection and corresponding control on parallel IGBT modules, and the current distribution of each branch cannot be known. If the IGBT branch is overcurrent, it will cause a single IGBT to be overloaded. Long-term use will greatly shorten the life of the IGBT itself, thus affecting the service life of the equipment. If the IGBT branch is seriously overcurrent, it will cause the IGBT to fail or be damaged, resulting in equipment damage and inability to continue treatment for the patient.

Technical Solution

The embodiments of the present invention provide a current detection device, a control method, and a vascular calcification treatment apparatus, in order to solve the problem in the prior art that the branch currents are seriously unbalanced, thereby causing abnormal equipment discharge and equipment damage.
To achieve the above object, the technical scheme of the present invention is implemented as follows:
In a first aspect, an embodiment of the present invention discloses a current detection device, the current detection device including:

a current detection module, arranged in a high-voltage discharge circuit, the current detection module being used for performing current detection on a first branch and a second branch of the high-voltage discharge circuit so as to respectively obtain a first current signal and a second current signal;
a processing module, coupled to the current detection module, wherein when a current difference between the first current signal and the second current signal is not within an allowable range, the processing module generates a discharge anomaly prompt signal;
a control module, connected to the processing module and the current detection module, used for, upon receiving the discharge anomaly prompt signal sent by the processing module, sending a pulse signal so as to disconnect the first branch and the second branch.

In an embodiment, the current detection device includes a differential amplification circuit, the differential amplification circuit connected between the current detection module and the processing module, and performing differential amplification on the first current signal and the second current signal.
In an embodiment, the current detection module includes:

a first PCB Rogowski coil, the first PCB Rogowski coil generating a first coil output voltage based on the first current signal;
a second PCB Rogowski coil, the second PCB Rogowski coil generating a second coil output voltage based on the second current signal;
wherein, based on a difference between the first coil output voltage and the second coil output voltage, it is determined whether the current difference between the first current signal and the second current signal is within an allowable range.

In an embodiment, the current detection device further includes:

a measured resistor, a first end of the measured resistor connected to a first end of the first PCB Rogowski coil, a second end of the measured resistor connected to a first end of the second PCB Rogowski coil, and a potential difference existing across the fist terminal and the second terminal of the measured resistor; wherein, a second terminal of the first PCB Rogowski coil is connected to a second terminal of the second PCB Rogowski coil, and winding directions of the first PCB Rogowski coil and the second PCB Rogowski coil are opposite, so that directions of the first coil output voltage and the second coil output voltage are opposite;

EP 4 667 947 A1

the control module respectively transmits the pulse signal to a control terminal of a second switching element and a control terminal of a first switching element via a fourth resistor and a fifth resistor; wherein, the first switching element is arranged in the first branch, a first terminal of the first switching element receives the first current signal via a first current-sharing resistor, the second switching element is arranged in the second branch, a first terminal of the second switching element receives the second current signal via a second current-sharing resistor, second terminals of the first switching element and the second switching element are both connected to a third resistor;

wherein, when the current difference between the first current signal and the second current signal is not within the allowable range, the pulse signal sent by the control module is used to disconnect the first switching element and the second switching element;

when the current difference between the first current signal and the second current signal is within the allowable range, the pulse signal sent by the control module after a preset duration is used to disconnect the first switching element and the second switching element;

when the current value of the first current signal is equal to that of the second current signal, the magnitudes of the first coil output voltage and the second coil output voltage are the same, and the current flowing through the measured resistor is zero;

when the current value of the first current signal is greater than that of the second current signal, the first coil output voltage is greater than the second coil output voltage, a first induced current is greater than a second induced current, the current flowing through the measured resistor is the first induced current minus the second induced current, and the potential at the first end of the measured resistor is higher than that at the second end;

when the current value of the first current signal is less than that of the second current signal, the first coil output voltage is less than the second coil output voltage, the first induced current is less than the second induced current, the current flowing through the measured resistor is the second induced current minus the first induced current, and the potential at the first end of the measured resistor is lower than that at the second end.

In an embodiment, the current detection apparatus includes a differential amplification circuit. The differential amplification circuit includes:

a first comparator, a first input terminal of the first comparator connected to a first terminal of the measured resistor, a second input terminal of the first comparator connected to a first terminal of a sixth resistor;

a second comparator, a first input terminal of the second comparator connected to a second terminal of the sixth resistor, a second input terminal of the second comparator connected to a second terminal of the measured resistor;

a first differential resistor, a first terminal of the first differential resistor connected to the first terminal of the sixth resistor and the second input terminal of the first comparator, a second terminal of the first differential resistor connected to an output terminal of the first comparator;

a second differential resistor, a first terminal of the second differential resistor connected to the second terminal of the sixth resistor and the first input terminal of the second comparator, a second terminal of the second differential resistor connected to an output terminal of the second comparator;

a third differential resistor, a first terminal of the third differential resistor connected to the second terminal of the first differential resistor and the output terminal of the first comparator;

a third comparator, a first input terminal of the third comparator connected to a second terminal of the third differential resistor;

a fourth differential resistor, a first terminal of the fourth differential resistor connected to the second terminal of the third differential resistor and the first input terminal of the third comparator, a second terminal of the fourth differential resistor connected to an output terminal of the third comparator;

a fifth differential resistor, a first terminal of the fifth differential resistor connected to the second terminal of the second differential resistor and the output terminal of the second comparator;

a sixth differential resistor, a first terminal of the sixth differential resistor connected to the second terminal of the fifth differential resistor and a second input terminal of the third comparator, a second terminal of the sixth differential resistor receiving a bias voltage signal;

wherein, the first differential resistor and the second differential resistor are identical, and the third differential resistor, the fourth differential resistor, the fifth differential resistor, and the sixth differential resistor are identical.

In an embodiment, the voltage difference between the output voltage of the first comparator and the output voltage of the second comparator is expressed as:

4

$$U_{dc} = \frac{U_{ba}}{R_G} * (R_G + R_{F1} + R_{F2})$$

When the bias voltage signal acts alone, the first output voltage at the output terminal of the third comparator is expressed as:

$$U_{out1} = U_{ref};$$

The second input terminal voltage of the third comparator is the divided voltage of the output voltage of the second comparator at the fifth differential resistor and the sixth differential resistor; the first input terminal voltage of the third comparator is the divided voltage of the second output voltage at the output terminal of the third comparator at the third differential resistor and the fourth differential resistor; when the output voltage of the second comparator acts alone:

$$\frac{U_d}{R_{F5} + R_{F6}} * R_{F6} = \frac{U_{out2}}{R_{F3} + R_{F4}} * R_{F3}$$

Then, the second output voltage at the output terminal of the third comparator is expressed as:

$$U_{out2} = U_d * \frac{R_{F3} + R_{F4}}{R_{F5} + R_{F6}} * \frac{R_{F3}}{R_{F6}}.$$

When the output voltage of the first comparator acts alone:

$$\frac{-U_c}{R_{F3} + R_{F4}} * R_{F4} = \frac{U_{out3}}{R_{F5} + R_{F6}} * R_{F6}$$

Then, the third output voltage at the output terminal of the third comparator is expressed as:

$$U_{out3} = -U_c * \frac{R_{F5} + R_{F6}}{R_{F3} + R_{F4}} * \frac{R_{F4}}{R_{F6}}.$$

The output voltage at the output terminal of the third comparator is equal to the sum of the first output voltage, the second output voltage, and the third output voltage at the output terminal of the third comparator, and is expressed as:

$$U_{out} = U_{out1} + U_{out2} + U_{out3} = U_{ref} + U_d * \frac{R_{F3} + R_{F4}}{R_{F5} + R_{F6}} * \frac{R_{F3}}{R_{F6}} - U_c * \frac{R_{F5} + R_{F6}}{R_{F3} + R_{F4}} * \frac{R_{F4}}{R_{F6}}.$$

$$U_{out} = U_{ref} + U_d - U_c = U_{ref} + \frac{U_{ba}}{R_G} * (R_G + R_{F1} + 1$$

wherein, the amplification factor G is:

$$G = 1 + \frac{R_{F1} + R_{F2}}{R_G}$$

In an embodiment, the processing module includes:
a processor connected to the control module; when the current difference between the first current signal and the second current signal is not within the allowable range, the processor controls the pulse signal sent by the control module to disconnect the first switching element and the second switching element.

In an embodiment, the processing module further includes:

a first digital-to-analog converter, a first terminal of the first digital-to-analog converter connected to the processor, a second terminal of the first digital-to-analog converter connected to the second terminal of the sixth differential resistor and providing the bias voltage signal;

a second digital-to-analog converter, a first terminal of the second digital-to-analog converter connected to the processor;

a fourth comparator, a first input terminal of the fourth comparator connected to the second terminal of the second digital-to-analog converter and receiving a first reference comparison voltage; a second input terminal of the fourth comparator connected to the output terminal of the third comparator and receiving the output voltage at the output terminal of the third comparator;

a third digital-to-analog converter, a first terminal of the third digital-to-analog converter connected to the processor;

a fifth comparator, a first input terminal of the fifth comparator connected to the output terminal of the third comparator and the second input terminal of the fourth comparator and receiving the output voltage at the output terminal of the third comparator; a second input terminal of the fifth comparator connected to the second terminal of the third digital-to-analog converter and receiving a second reference comparison voltage.

In an embodiment, the processing module further includes:

a first diode, a first terminal of the first diode connected to the output terminal of the fourth comparator;

a second diode, a first terminal of the second diode connected to the output terminal of the fifth comparator;

a first bistable trigger, a pulse signal terminal of the first bistable trigger connected to the second terminal of the first diode, a preset level signal terminal, an input terminal, a clear signal terminal and an output terminal of the first bistable trigger connected to the processor;

a second bistable trigger, a pulse signal terminal of the second bistable trigger connected to the second terminal of the second diode, a preset level signal terminal, an input terminal, a clear signal terminal and an output terminal of the second bistable trigger connected to the processor;

wherein, when the current value of the first current signal is greater than the current value of the second current signal and the difference is not within the allowable range, the output voltage at the output terminal of the third comparator is lower than the first reference comparison voltage, the output terminal of the fourth comparator is at a low level, the output terminal of the fifth comparator is at a high level, the output terminal of the first bistable trigger is at a low level, the output terminal of the second bistable trigger is at a high level, and the processor sends the discharge anomaly prompt signal to the control module and disconnects the first switching element and the second switching element;

wherein, when the current value of the first current signal and the current value of the second current signal are within the allowable range, the output voltage at the output terminal of the third comparator is between the first reference comparison voltage and the second reference comparison voltage, the output terminal of the fourth comparator is at a high level, the output terminal of the fifth comparator is at a high level, the output terminal of the first bistable trigger is at a high level, the output terminal of the second bistable trigger is at a high level, and the processor does not send the discharge anomaly prompt signal to the control module;

wherein, when the current value of the first current signal is less than the current value of the second current signal and the difference is not within the allowable range, the output voltage at the output terminal of the third comparator is higher than the second reference comparison voltage, the output terminal of the fourth comparator is at a high level, the output terminal of the fifth comparator is at a low level, the output terminal of the first bistable trigger is at a high level, the output terminal of the second bistable trigger QD2 is at a low level, and the processor sends the discharge anomaly prompt signal to the control module and disconnects the first switching element and the second switching element.

In a second aspect, an embodiment of the present invention further discloses a control method for the current detection device, the control method being applied to the above-described current detection device, the control method including:

performing current detection on a first branch and a second branch of a high-voltage discharge circuit so as to respectively obtain a first current signal and a second current signal;

when the current difference between the first current signal and the second current signal is not within the allowable range, generating a discharge anomaly prompt signal;

sending a pulse signal based on the discharge anomaly prompt signal so as to disconnect the first branch and the second branch.

In a third aspect, the present invention provides a vascular calcification treatment apparatus, including the current detection device as above, and a solution tank; wherein two ends of the solution tank are respectively connected to positive and negative high voltages so as to form a high-voltage discharge circuit.

Beneficial Effects

It can be used for avoiding serious current imbalance between the first branch and the second branch connected in parallel in the high-voltage discharge circuit, thereby improving system stability and equipment safety. In other words, the current detection device proposed by the present invention is provided with a processor which can monitor and handle the over-current issue during discharge in the first and second branches. At the same time, by presetting thresholds through the first, second and third digital-to-analog converters (bias voltage signal, first reference comparison voltage, second reference comparison voltage), there is no need to concern oneself with the discharge process; only the electrical interface level state of the processor needs to be read to make a judgment. The presetting of the thresholds allows system parameter errors to exist during product production and testing, giving high flexibility. In addition, the current detection module can be equipped with PCB Rogowski coils, which are electrically isolated from the measured conductors (first and second branches), do not consume primary-side energy during measurement, and do not alter the original electrical circuit due to non-contact measurement. Moreover, the absence of a core structure avoids magnetic saturation issues, and PCB Rogowski coils feature high manufacturing precision, good consistency, low production cost, and do not need to be connected to the high-voltage discharge circuit, avoiding the impact of the high-voltage circuit on the measured circuit, thereby ensuring high safety.

BRIEF DESCRIPTION OF THE DRAWINGS

FIG. 1 is a circuit schematic diagram of the current detection device provided by an embodiment of the present invention.

FIG. 2 is an equivalent circuit diagram of the first PCB Rogowski coil and the second PCB Rogowski coil in the current detection device provided by an embodiment of the present invention.

FIG. 3 is a flowchart of the control method of the current detection device provided by an embodiment of the present invention.

FIG. 4 is a detailed flowchart of the control method of the current detection device provided by an embodiment of the present invention.

FIG. 5 is a circuit schematic diagram of the vascular calcification treatment apparatus provided by an embodiment of the present invention.

DETAILED DESCRIPTION OF THE EMBODIMENTS

Below, specific embodiments of the present invention will be described in detail in conjunction with the accompanying drawings. Obviously, the described embodiments are only part of the embodiments of the present invention, rather than all of them. Based on the description of the present invention, all other embodiments obtained by those of ordinary skill in the art without making creative efforts fall within the scope of protection of the present invention.

In the description of the present invention, unless otherwise expressly specified and defined, terms such as "arranged", "installed", and "connected" shall be understood in a broad sense; for example, they may be a fixed connection, a detachable connection, or an integral connection; they may be a mechanical connection or an electrical connection; they may be directly connected or indirectly connected through an intermediate medium. For those of ordinary skill in the art, the specific meanings of the above terms can be understood according to specific circumstances.

Terms such as "first", "second", "third" and the like are merely used to distinguish between similar values or components, and do not indicate or imply relative importance or a specific order.

The terms "include", "comprise" or any other variant thereof are intended to cover a non-exclusive inclusion, such that not only the listed elements are included, but also other elements not expressly listed may be included.

**EP 4 667 947 A1**

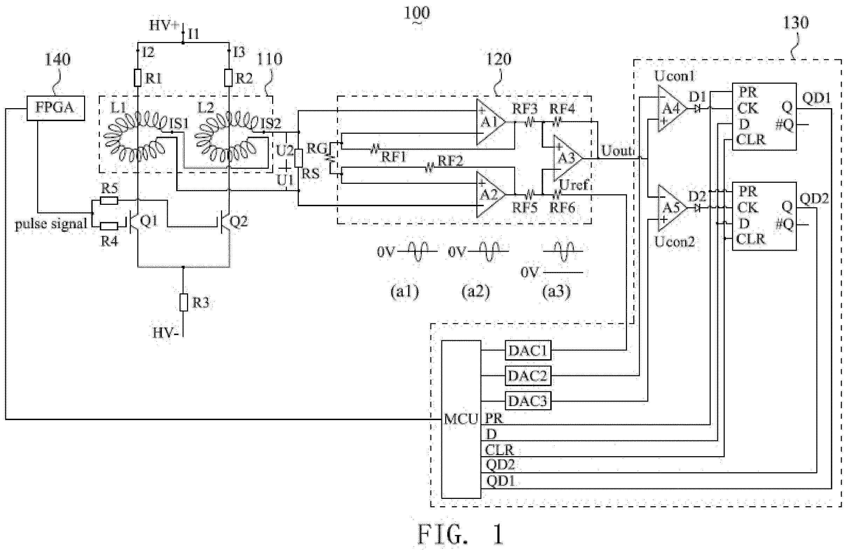

Please refer to FIG. 1. FIG. 1 is a circuit schematic diagram of the current detection device provided by an embodiment of the present invention. The current detection device 100 includes a current detection module 110, a processing module 130, and a control module 140. The current detection module 110 is arranged in a high-voltage discharge circuit and is used for performing current detection on a first branch and a second branch of the high-voltage discharge circuit, so as to respectively obtain a first current signal $I_2$ and a second current signal $I_3$. The processing module 130 is coupled to the current detection module 110; when the current difference between the first current signal $I_2$ and the second current signal $I_3$ is not within an allowable range, the processing module 130 generates a discharge anomaly prompt signal. The control module 140 is connected to the processing module 130 and the current detection module 110 and is used for, upon receiving the discharge anomaly prompt signal sent by the processing module 130, sending a pulse signal so as to disconnect the first branch and the second branch.

In an embodiment, as shown in FIG. 1, the current detection device includes a differential amplification circuit 120, connected between the current detection module 110 and the processing module 130, performing differential amplification on the first current signal $I_2$ and the second current signal $I_3$.

In an embodiment, as shown in FIG. 1, the current detection module 110 includes a first PCB Rogowski coil $L_1$ and a second PCB Rogowski coil $L_2$. The first PCB Rogowski coil $L_1$ generates a first coil output voltage $U_1$ based on the first current signal $I_2$. The second PCB Rogowski coil $L_2$ generates a second coil output voltage $U_2$ based on the second current signal $I_3$. Based on the difference between the first coil output voltage $U_1$ and the second coil output voltage $U_2$, it is determined whether the current difference between the first current signal $I_2$ and the second current signal $I_3$ is within the allowable range.

In an embodiment, as shown in FIG. 1, the current detection device 100 further includes a measured resistor RS; a first terminal of the measured resistor RS is connected to a first terminal of the first PCB Rogowski coil $L_1$, a second terminal of the measured resistor RS is connected to a first terminal of the second PCB Rogowski coil $L_2$, and a potential difference Uba exists across the two terminals of the measured resistor RS; the second terminal of the first PCB Rogowski coil $L_1$ is connected to the second terminal of the second PCB Rogowski coil $L_2$, and the winding directions of the first PCB Rogowski coil $L_1$ and the second PCB Rogowski coil $L_2$ are opposite, so that the directions of the first coil output voltage $U_1$ and the second coil output voltage $U_2$ are opposite.

The control module 140 respectively transmits the pulse signal to the control terminal of the second switching element $Q_2$ and the control terminal of the first switching element $Q_1$ via a fourth resistor $R_4$ and a fifth resistor $R_5$. The first switching element $Q_1$ is arranged in the first branch, a first terminal of the first switching element $Q_1$ receives the first current signal $I_2$ via a first current-sharing resistor $R_1$, the second switching element $Q_2$ is arranged in the second branch, a first terminal of the second switching element $Q_2$ receives the second current signal $I_3$ via a second current-sharing resistor $R_2$, and second terminals of the first switching element $Q_1$ and the second switching element $Q_2$ are both connected to a third resistor $R_3$.

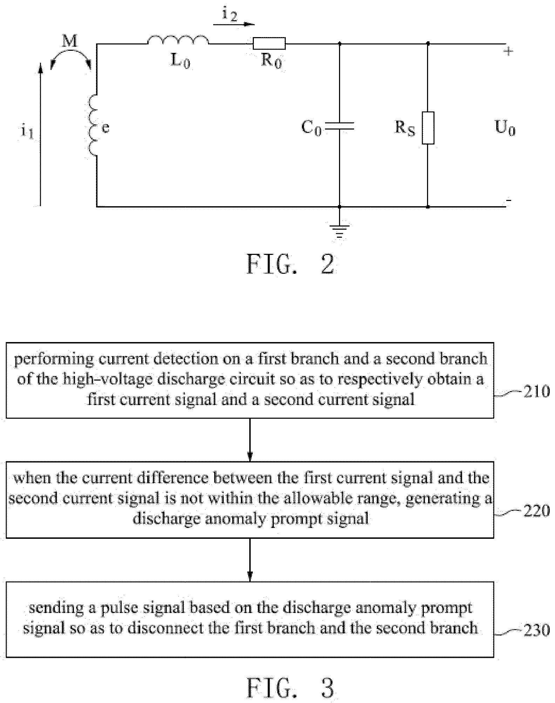

FIG. 2 is an equivalent circuit diagram of the first PCB Rogowski coil and the second PCB Rogowski coil in the current detection device provided by an embodiment of the present invention. When the PCB Rogowski coil measures current, the coil output terminal needs to be connected to a sampling resistor; the equivalent circuit is as shown in FIG. 2, wherein $L_0$ is the self-inductance of the PCB Rogowski coil, $R_0$ is the internal resistance of the PCB Rogowski coil, Co is the distributed capacitance, RS is the sampling resistor; $i_1$ is the measured current, $i_2$ is the induced current of the coil, $U_0$ is the output voltage of the coil, and e is the induced electromotive force of the coil, which can be integrated by an integration circuit, and the output voltage $U_0$ of the coil is proportional to the measured current $i_1$. The sensing principle of the PCB Rogowski coil is the principle of electromagnetic induction, which features small size and low cost, and the relationship between the signal output and the measured current intensity is very linear. Due to electrical isolation between the PCB Rogowski coil and the measured conductor, no primary-side energy is consumed during measurement. Moreover, the absence of a core structure avoids magnetic saturation issues. Pulsed large currents have large peaks, fast rising edges, and wide frequency coverage, requiring measurement sensors with short time and wide bandwidth. The change in magnetic flux is the change in current. Rogowski coils were originally used to measure pulsed large currents; with technological development, the accuracy and response time of Rogowski coils for measuring pulsed large currents have been greatly improved. The PCB coil is fabricated on a PCB board, featuring high manufacturing precision, good consistency, suitability for mass production, and low mass-production cost.

Specifically, the first switching element $Q_1$ receives the first current $I_2$ of the first branch of the high-voltage discharge circuit via the first current-sharing resistor $R_1$. The second switching element $Q_2$ receives the second current $I_3$ of the second branch of the high-voltage discharge circuit via the second current-sharing resistor $R_2$, and the first current-sharing resistor $R_1$ and the second current-sharing resistor $R_2$ are connected to the positive high voltage HV$^+$. Wherein, $I_1$ = $I_2$ + $I_3$. The first current signal $I_2$ is used to generate the first coil output voltage $U_1$ in the first PCB Rogowski coil $L_1$. The second current signal $I_3$ is used to generate the second coil output voltage $U_2$ in the second PCB Rogowski coil $L_2$. The second switching element $Q_2$ is connected in parallel with the first switching element $Q_1$. The second terminals of the first switching element $Q_1$ and the second switching element $Q_2$ are connected to a first terminal of the third resistor $R_3$, and the third resistor $R_3$ is connected to the negative high voltage HV$^-$.

The processing module 130 is coupled to the current detection module 110; when the current difference between the first current signal $I_2$ and the second current signal $I_3$ is not within the allowable range, the processing module 130 generates a discharge anomaly prompt signal. The control module 140 is connected to the processing module 130 and the current detection module 110 and is used for, upon receiving the discharge anomaly prompt signal sent by the processing module 130, sending a pulse signal so as to disconnect the first branch and the second branch.

Wherein, when the current difference between the first current signal $I_2$ and the second current signal $I_3$ is not within the allowable range, the pulse signal sent by the control module 140 is used to disconnect the first switching element $Q_1$ and the second switching element $Q_2$;

when the current difference between the first current signal $I_2$ and the second current signal $I_3$ is within the allowable range, the pulse signal sent by the control module 140 after reaching a preset duration is used to disconnect the first switching element $Q_1$ and the second switching element $Q_2$;

when the current value of the first current signal $I_2$ is equal to the current value of the second current signal $I_3$, the magnitudes of the first coil output voltage $U_1$ and the second coil output voltage $U_2$ are the same, and the current flowing through the measured resistor RS is zero;

when the current value of the first current signal $I_2$ is greater than the current value of the second current signal $I_3$, the first coil output voltage $U_1$ is greater than the second coil output voltage $U_2$, the first induced current IS1 is greater than the second induced current IS2, the current flowing through the measured resistor RS is the first induced current IS1 minus the second induced current IS2, and the first terminal potential Ua of the measured resistor RS, is higher than the second terminal potential, Ub;

when the current value of the first current signal $I_2$ is less than the current value of the second current signal $I_3$, the first coil output voltage $U_1$ is less than the second coil output voltage $U_2$, the first induced current IS1 is less than the second induced current IS2, the current flowing through the measured resistor RS is the second induced current IS2 minus the first induced current IS1, and the first terminal potential Ua of the measured resistor RS, is lower than the second terminal potential Ub.

Thus, when the current difference between the first current signal $I_2$ and the second current signal $I_3$ is not within the allowable range, the pulse signal sent by the control module 140 such as a field-programmable gate array circuit FPGA is used to disconnect the first switching element $Q_1$ and the second switching element $Q_2$. On the other hand, when the current difference between the first current signal $I_2$ and the second current signal $I_3$ is within the allowable range, the pulse signal sent by the control module 140 such as a field-programmable gate array circuit FPGA is used to close the first switching element $Q_1$ and the second switching element $Q_2$. The current detection device 100 provided by the present invention allows preset tolerance for system parameters by presetting thresholds, and then disconnects or closes the first switching element $Q_1$ and the second switching element $Q_2$ such as transistors, thereby featuring high flexibility and high precision in automatic circuit control.

In other words, the current detection device 100 proposed in the present application uses the first branch and the second branch in parallel, to share the large current in the high-voltage discharge circuit, increasing the system's ability to withstand load current and improving system stability, thereby offering high cost-effectiveness. However, due to internal parameter differences of switching elements such as IGBT on each branch and asymmetries in circuit topology and driver-circuit layout, the currents output by the parallel branches are difficult to maintain completely identical, which brings about both static current-unbalance issue and dynamic current-unbalance issue for the switching elements such as IGBT on each parallel branch. The static current unbalance mainly refers to the uneven distribution of load current among the branches where the switching elements such as IGBT are located when the switching elements such as IGBT on each parallel branch are in the on-state. It usually originates from inconsistencies in the following factors of each switching element such as IGBT: output characteristics of the switching element such as IGBT, drive voltage applied to the gate of the switching element such as IGBT, power-loop characteristics of the branch where each parallel switching element such as IGBT is located, and temperature characteristics. The dynamic current unbalance mainly refers to the inconsistency in the start moment of the turn-on and turn-off processes of each parallel switching element such as IGBT and the corresponding rate of change of the collector-gate current during these processes, causing some switching elements such as IGBT to bear larger currents. Factors triggering the dynamic current unbalance typically include: switching characteristics of the switching element such as IGBT, gate-drive-loop parameters, power-loop characteristics, and temperature characteristics. With long-term use of the switching elements such as IGBT, those bearing larger currents will prematurely age or fail, affecting system reliability and stability. If branch currents are detected in each branch of the high-voltage discharge, the current conditions of each switching element such as IGBT during the discharge process can be understood; when the branch-current difference reaches a set value, the detection output state changes, the processing module 130 acquires the change and issues a prompt to inform the user of the equipment discharge status, and, when necessary, terminates the discharge process and issues an abnormal-alarm prompt.

In an embodiment, the current detection device 100 further includes the differential amplification circuit 120. The differential amplification circuit 120 includes the first comparator $A_1$, the second comparator $A_2$, the third comparator $A_3$, the first differential resistor $RF_1$, the second differential resistor $RF_2$, the third differential resistor $RF_3$, the fourth differential resistor $RF_4$, the fifth differential resistor $RF_5$ and the sixth differential resistor $RF_6$. A first input terminal of the first comparator $A_1$ is connected to a first terminal of the measured resistor RS. A second input terminal of the first comparator $A_1$ is connected to a first terminal of the sixth resistor RG. A first input terminal of the second comparator $A_2$ is connected to a second terminal of the sixth resistor RG. A second input terminal of the second comparator $A_2$ is connected to a second terminal of the measured resistor RS. A first terminal of the first differential resistor $RF_1$ is connected to the first terminal of the sixth resistor RG and the second input terminal of the first comparator $A_1$. A second terminal of the first differential resistor $RF_1$ is connected to an output terminal of the first comparator $A_1$. A first terminal of the second differential resistor $RF_2$ is connected to the second terminal of the sixth resistor RG and the first input terminal of the second comparator $A_2$. A second terminal of the second differential resistor $RF_2$ is connected to an output terminal of the second comparator $A_2$. A first terminal of the third differential resistor $RF_3$ is connected to the second terminal of the first differential resistor $RF_1$ and the output terminal of the first comparator $A_1$. A first input terminal of the third comparator $A_3$ is connected to a second terminal of the third differential resistor $RF_3$. A first terminal of the fourth differential resistor $RF_4$ is connected to the second terminal of the third differential resistor $RF_3$ and the first input terminal of the third comparator $A_3$. A second terminal of the fourth differential resistor $RF_4$ is connected to an output terminal of the third comparator $A_3$. A first terminal of the fifth differential resistor $RF_5$ is connected to the second terminal of the second differential resistor $RF_2$ and the output terminal of the second comparator $A_2$. A first terminal of the sixth differential resistor $RF_6$ is connected to the second terminal of the fifth differential resistor $RF_5$ and the second input terminal of the third comparator $A_3$. A second terminal of the sixth differential resistor $RF_6$ receives the bias voltage signal Uref. In this embodiment, the first differential resistor $RF_1$ and the second differential resistor $RF_2$ are set to be identical, and the third differential resistor $RF_3$, the fourth differential resistor $RF_4$, the fifth differential resistor $RF_5$ and the sixth differential resistor $RF_6$ are set to be identical, so as to facilitate subsequent formula derivation, to allow system parameter errors during testing by presetting thresholds, to provide high flexibility and to increase circuit control precision.

In an embodiment, the voltage difference Udc between the output voltage Uc of the first comparator $A_1$ and the output voltage Ud of the second comparator $A_2$ is expressed as:

$$U_{dc} = \frac{U_{ba}}{R_G} * (R_G + R_{F1} + R_{F2});$$

When the bias voltage signal Uref acts alone, the first output voltage Uout1 at the output terminal of the third comparator $A_3$ is expressed as:

$$U_{out1} = U_{ref};$$

The second input terminal voltage Uf of the third comparator $A_3$ is the divided voltage of the output voltage Ud of the second comparator $A_2$ at the fifth differential resistor $RF_5$ and the sixth differential resistor $RF_6$; the first input terminal voltage Ue of the third comparator $A_3$ is the divided voltage of the second output voltage Uout2 at the output terminal of the third comparator $A_3$ at the third differential resistor $RF_3$ and the fourth differential resistor $RF_4$; when the output voltage Ud of the second comparator $A_2$ acts alone,

$$\frac{U_d}{R_{F5} + R_{F6}} * R_{F6} = \frac{U_{out2}}{R_{F3} + R_{F4}} * R_{F3};$$

Then, the second output voltage Uout2 at the output terminal of the third comparator $A_3$ is expressed as:

$$U_{out2} = U_d * \frac{R_{F3} + R_{F4}}{R_{F5} + R_{F6}} * \frac{R_{F3}}{R_{F6}};$$

When the output voltage Uc of the first comparator $A_1$ acts alone,

$$\frac{-U_c}{R_{F3} + R_{F4}} * R_{F4} = \frac{U_{out3}}{R_{F5} + R_{F6}} * R_{F6};$$

Then, the third output voltage Uout3 at the output terminal of the third comparator $A_3$ is expressed as:

$$U_{out3} = -U_c * \frac{R_{F5} + R_{F6}}{R_{F3} + R_{F4}} * \frac{R_{F4}}{R_{F6}};$$

The output voltage Uout at the output terminal of the third comparator $A_3$ is equal to the sum of the first output voltage Uout1, the second output voltage Uout2 and the third output voltage Uout3 at the output terminal of the third comparator $A_3$, and is expressed as:

$$U_{out} = U_{out1} + U_{out2} + U_{out3} = U_{ref} + U_d * \frac{R_{F3} + R_{F4}}{R_{F5} + R_{F6}} * \frac{R_{F3}}{R_{F6}} - U_c * \frac{R_{F5} + R_{F6}}{R_{F3} + R_{F4}} * \frac{R_{F4}}{R_{F6}}.$$

$$U_{out} = U_{ref} + U_d - U_c = U_{ref} + \frac{U_{ba}}{R_G} * (R_G + R_{F1} + I;$$

Wherein, the amplification factor G is:

$$G = 1 + \frac{R_{F1} + R_{F2}}{R_G};$$

In an embodiment, the processing module 130 further includes the processor MCU, connected to the control module 140; when the current difference between the first current $I_2$ and the second current signal $I_3$ is not within the allowable range, the processor MCU controls the pulse signal sent by the control module 140 to disconnect the first switching element $Q_1$ and the second switching element $Q_2$.

In an embodiment, the processing module 130 further includes the first digital-to-analog converter DAC1, the second digital-to-analog converter DAC2, the third digital-to-analog converter DAC3, the fourth comparator $A_4$, and the fifth comparator As. The processor MCU is connected to the field-programmable gate array circuit FPGA. When the current difference between the first current $I_2$ and the second current signal $I_3$ is not within the allowable range, the processor MCU controls the field-programmable gate array circuit FPGA to send the pulse signal to disconnect the first switching element $Q_1$ and the second switching element $Q_2$, so as to prevent damage to the equipment caused by overcurrent. A first terminal of the first digital-to-analog converter DAC1 is connected to the processor MCU. A second terminal of the first digital-to-analog converter DAC1 is connected to the second terminal of the sixth differential resistor $RF_6$ and provides the bias voltage signal Uref. A first terminal of the second digital-to-analog converter DAC2 is connected to the processor MCU. A first input terminal of the fourth comparator $A_4$ is connected to the second terminal of the second digital-to-analog converter DAC2 and receives the first reference comparison voltage Ucon1. A second input terminal of the fourth comparator $A_4$ is connected to the output terminal of the third comparator $A_3$ and receives the output voltage Uout at the output terminal of the third comparator $A_3$. A first terminal of the third digital-to-analog converter DAC3 is connected to the processor MCU. A first input terminal of the fifth comparator As is connected to the output terminal of the third comparator $A_3$ and the second input terminal of the fourth comparator $A_4$ and receives the output voltage Uout at the output terminal of the third comparator $A_3$. A second input terminal of the fifth comparator As is connected to the second terminal of the third digital-to-analog converter DAC3 and receives the second reference comparison voltage Ucon2.

In an embodiment, the current detection device 100 further includes the first diode $D_1$, the second diode $D_2$, the

first bistable trigger $QD_1$ and the second bistable trigger $QD_2$. A first terminal of the first diode $D_1$ is connected to the output terminal of the fourth comparator $A_4$. A first terminal of the second diode $D_2$ is connected to the output terminal of the fifth comparator $A_5$. A clock input terminal CK of the first bistable trigger $QD_1$ is connected to the second terminal of the first diode $D_1$. A preset terminal PR, a data input terminal D, a clear terminal CLR and an output terminal Q of the first bistable trigger $QD_1$ are connected to the processor MCU. A clock input terminal CK of the second bistable trigger $QD_2$ is connected to the second terminal of the second diode $D_2$. A preset terminal PR, a data input terminal D, a clear terminal CLR and an output terminal Q of the second bistable trigger $QD_2$ are connected to the processor MCU.

Specifically, when the current value of the first current signal $I_2$ is greater than the current difference value of the second current signal $I_3$ and the difference is not within the allowable range, the output voltage Uout at the output terminal of the third comparator $A_3$ is lower than the first reference comparison voltage Ucon1, the output terminal of the fourth comparator $A_4$ is at a low level, the output terminal of the fifth comparator As is at a high level, the output terminal of the first bistable trigger $QD_1$ is at a low level, the output terminal of the second bistable trigger $QD_2$ is at a high level, and the processor MCU sends the discharge anomaly prompt signal to the field-programmable gate array circuit FPGA and disconnects the first switching element $Q_1$ and the second switching element $Q_2$.

In an embodiment, when the current value of the first current signal $I_2$ and the current difference value of the second current signal $I_3$ are within the allowable range, the output voltage Uout at the output terminal of the third comparator $A_3$ is between the first reference comparison voltage Ucon1 and the second reference comparison voltage Ucon2, the output terminal of the fourth comparator $A_4$ is at a high level, the output terminal of the fifth comparator As is at a high level, the output terminal of the first bistable trigger $QD_1$ is at a high level, the output terminal of the second bistable trigger $QD_2$ is at a high level, and the processor MCU controls the field-programmable gate array circuit FPGA to send the pulse signal so as to close the first switching element $Q_1$ and the second switching element $Q_2$.

In an embodiment, when the current value of the first current signal $I_2$ is less than the current difference value of the second current signal $I_3$ and the difference is not within the allowable range, the output voltage Uout at the output terminal of the third comparator $A_3$ is higher than the second reference comparison voltage Ucon2. The output terminal of the fourth comparator $A_4$ is at a high level, the output terminal of the fifth comparator As is at a low level, the output terminal of the first bistable trigger $QD_1$ is at a high level, the output terminal of the second bistable trigger $QD_2$ is at a low level, and the processor MCU sends the discharge anomaly prompt signal value to the field-programmable gate array circuit FPGA and disconnects the first switching element $Q_1$ and the second switching element $Q_2$. The current detection device 100 of the present invention simultaneously uses preset thresholds of the first, second and third digital-to-analog converter circuits; there is no need to concern oneself with excessive errors generated during the discharge process, and judgment can be made simply by reading the level state of the electrical interface of the processor, thereby providing high flexibility and high precision in automatic control.

Thus, the current detection device 100 proposed in the present application can be used for branch current sharing detection. By detecting the branch currents on each branch of the high-voltage discharge circuit and performing differential amplification on the signals collected from each branch, the output amplitude becomes larger. After biasing, the output voltages are all positive. The processing module 130 sets the upper and lower limit voltages for the current difference via the DAC module; the resulting voltage is then compared by a comparator and the level is held by a bistable trigger. If the differential-amplified and biased voltage is within the upper and lower limits, the levels output by the bistable triggers are all high, indicating that the branch current difference is within the allowable range, the currents of the two branches are not significantly different, and the branch currents are within the withstand range of switching elements such as IGBTs, eliminating the risk of overcurrent. If the differential-amplified and biased voltage is outside the upper and lower limits, the levels output by the first bistable trigger $QD_1$ and the second bistable trigger $QD_2$ contain a low level, indicating that the branch current difference is excessive, either the first branch or the second branch is overcurrent, and prolonged use will greatly shorten the service life of the switching elements such as IGBTs on that branch, or even cause failure and damage. The branch experiencing overcurrent can be identified by the pin with the low level. To prevent switching elements such as IGBTs from overcurrent due to large current differences, which would inevitably lead to failure or damage, upon detecting an abnormal pin level signal, the processing module 130 immediately sends a switch-off command to the control module 140 such as FPGA, and the control module 140 such as FPGA turns off the switching elements such as IGBTs to stop the high-voltage pulse output, thereby avoiding equipment damage.

Please refer to FIG. 3, which is a flowchart of the control method of the current detection device provided by an embodiment of the present invention. The embodiment of the present invention further provides a control method applied to the above current detection device; its specific implementation may refer to the corresponding content of the current detection device in the above embodiments. The control method includes:

S210, performing current detection on a first branch and a second branch of the high-voltage discharge circuit so as to respectively obtain a first current signal and a second current signal;
S220, when the current difference between the first current signal and the second current signal is not within the allowable range, generating a discharge anomaly prompt signal;

S230, sending a pulse signal based on the discharge anomaly prompt signal so as to disconnect the first branch and the second branch.

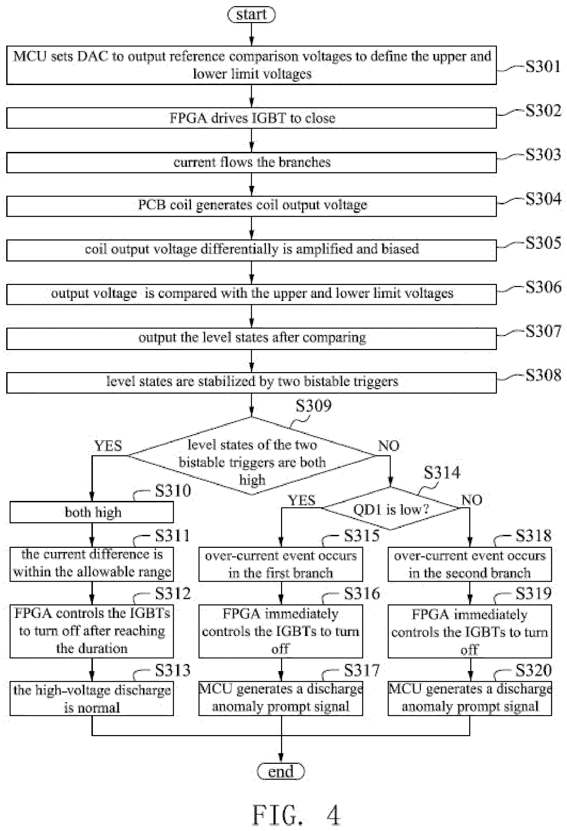

Please simultaneously refer to FIG. 1 and FIG. 4. FIG. 4 is a detailed flowchart of the control method of the current detection device provided by an embodiment of the present invention. The specific control method of an embodiment is as follows:

S301, the processor MCU sets the digital-to-analog converter DAC to output reference comparison voltages to define the upper and lower limit voltages;

S302, the field-programmable gate array circuit FPGA drives the first switching element $Q_1$ and the second switching element $Q_2$ such as IGBTs to close;

S303, the first current signal $I_2$ flows through the first branch, and the second current signal $I_3$ flows through the second branch;

S304, the first PCB Rogowski coil of the first branch generates a first induced electromotive force, or the second PCB Rogowski coil of the second branch generates a second induced electromotive force;

S305, the first coil output voltage $U_1$ and the second coil output voltage $U_2$ are differentially amplified and biased;

S306, the output voltage at the output terminal of the third comparator $A_3$ is compared with the upper and lower limit voltages;

S307, the level states at the output terminals of the first bistable trigger $QD_1$ and the second bistable trigger $QD_2$ are compared;

S308, the level states of the first bistable trigger $QD_1$ and the second bistable trigger $QD_2$ are stabilized;

S309, determining whether the level states at the output terminals of the first bistable trigger $QD_1$ and the second bistable trigger $QD_2$ are both high; if yes, executing S310, if not, executing S314;

S310, the level states at the output terminals of the first bistable trigger $QD_1$ and the second bistable trigger $QD_2$ are both high;

S311, the current difference between the first current signal $I_2$ and the second current signal $I_3$ is within the allowable range;

S312, the field-programmable gate array circuit controls the first switching element $Q_1$ and the second switching element $Q_2$ such as IGBTs to turn off after reaching the duration;

S313, the high-voltage discharge of the current detection device is normal, end;

S314, determining whether the level state at the output terminal of the first bistable trigger $QD_1$ is low;

S315, when the first bistable trigger $QD_1$ is at a low level, an over-current event occurs in the first branch through which the first current signal $I_2$ flows;

S316, the field-programmable gate array circuit FPGA immediately turns off the first and second switching elements;

S317, the processor MCU generates a discharge anomaly prompt signal based on the over-current event in the first branch, end;

S318, when the first bistable trigger is not at a low level, an over-current event occurs in the second branch through which the second current signal $I_2$ flows;

S319, the field-programmable gate array circuit FPGA immediately turns off the first switching element $Q_1$ and the second switching element $Q_2$ such as IGBTs;

S320, the processor MCU generates a discharge anomaly prompt signal based on the over-current event in the second branch, end.

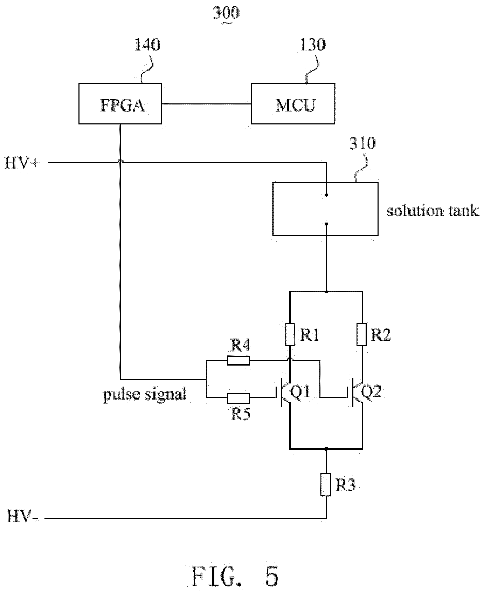

Please simultaneously refer to FIG. 1 and FIG. 5. FIG. 5 is a circuit schematic diagram of the vascular calcification treatment apparatus provided by an embodiment of the present invention. The present invention provides a vascular calcification treatment apparatus 300 including the above current detection device 100 and a solution tank 310. Two ends of the solution tank 310 are respectively connected to positive and negative high voltages HV+, HV- so as to form a high-voltage discharge circuit. HV+ and HV- have voltages up to several kilovolts; when the control module such as FPGA controls the parallel switching elements to conduct, there is an extremely large voltage difference across the solution G, the solution G will undergo ionization breakdown, during which the equivalent impedance of the solution is small, an extremely large current is generated in the discharge circuit, and this current flows through the two parallel switching elements $Q_1$, $Q_2$ and then through HV-. Due to the mismatch between application requirements and the inherent characteristics of conventional switching elements such as IGBTs, the current that the switching elements such as IGBTs can withstand is limited; a single switching element such as IGBT cannot withstand the current surge generated during discharge. Using switching elements in parallel can jointly carry the discharge current. Under relatively ideal conditions, the two branches equally share the current in the loop, so the current carried by each IGBT remains within the device's allowable range and will not cause IGBT damage. However, in actual use, due to differences among IGBTs and circuit topology, it is difficult to maintain complete consistency, and branch currents differ, so the current carried by each IGBT also differs.

This embodiment aims to detect the currents of the various parallel branches in the high-voltage discharge circuit of the vascular calcification treatment apparatus. By equipping each branch of the discharge circuit with corresponding PCB Rogowski coils, and differentially amplifying the output signals of the PCB Rogowski coils, the current differences among the branches can be obtained to perform current-sharing analysis of each branch. During the high-voltage discharge of the shock-wave equipment, there is an extremely large current flows through the high-voltage discharge circuit, i.e., the parallel branches will carry huge currents. If PCB Rogowski coils are respectively added to each branch, when current flows through a branch, the magnetic flux of the PCB Rogowski coil changes, and the PCB Rogowski coil generates a corresponding coil output voltage; the larger the current flowing through the branch, the stronger the magnetic induction and the higher the coil output voltage generated. Therefore, the magnitude of the current flowing through the switching elements such as IGBTs on the branch can be presented on the PCB coil output voltage. If the coil output voltages of the two branches are superimposed in reverse directions and processed by differential amplification-i.e., the waveform a1 in FIG. 1 becomes the waveform a2-and then biased so that the overall output voltage waveform is shifted upward, i.e., the waveform a3 in FIG. 1, voltages below 0 V do not appear, reducing detection circuit complexity and facilitating analysis and comparison. Then the output is fed into the comparison circuit. The processor MCU in the processing module 130 sets the upper and lower limit voltages of the comparison reference via the digital-to-analog converter module; comparison yields high or low levels, which are then fed into the bistable trigger QD to maintain the level, facilitating reading by the processor MCU via the processing module 130. Based on the states of the two output levels, whether the branch current is over-current and which branch is over-current can be determined. The current-sharing detection scheme compares the detected current difference with the set value; if the detected branch current difference is within the set range, it indicates that the currents of the two branches are similar and the discharge is as expected. If the detected branch current difference is outside the allowable range, it indicates that the branch current difference is large and one branch is over-current. At this time, the control module 140 will prohibit high-voltage pulse output and issue an abnormal prompt to prevent equipment damage and other safety accidents.

The current detection device, control method and vascular calcification treatment apparatus provided by the embodiments of the present invention: the current detection device includes a current detection module, a processing module and a control module; the current detection module is arranged in a high-voltage discharge circuit and is used for performing current detection on a first branch and a second branch of the high-voltage discharge circuit so as to respectively obtain a first current signal and a second current signal; the processing module is coupled to the current detection module, and when the current difference between the first current signal and the second current signal is not within the allowable range, the processing module generates a discharge anomaly prompt signal; the control module is connected to the processing module and the current detection module and is used for, upon receiving the discharge anomaly prompt signal sent by the processing module, sending a pulse signal so as to disconnect the first branch and the second branch. The present invention can be used for avoiding serious current imbalance between the first branch and the second branch connected in parallel in the high-voltage discharge circuit, thereby improving system stability and equipment safety. In other words, the current detection device proposed by the present invention is provided with a processor which can monitor and handle the over-current issue during discharge in the first and second branches. At the same time, by presetting thresholds through the first, second and third digital-to-analog converters (bias voltage signal, first reference comparison voltage, second reference comparison voltage), there is no need to concern oneself with the discharge process; only the electrical interface level state of the processor needs to be read to make a judgment. The presetting of the thresholds allows system parameter errors to exist during product production and testing, giving high flexibility. In addition, the current detection module can be equipped with PCB Rogowski coils, which are electrically isolated from the measured conductors (first and second branches), do not consume primary-side energy during measurement, and do not alter the original electrical circuit due to non-contact measurement. Moreover, the absence of a core structure avoids magnetic saturation issues, and PCB Rogowski coils feature high manufacturing precision, good consistency, low production cost, and do not need to be connected to the high-voltage discharge circuit, avoiding the impact of the high-voltage circuit on the measured circuit, thereby ensuring high safety.

The above is only the specific implementation of the present invention; however, the protection scope of the present invention is not limited thereto. Any person skilled in the art who makes changes or substitutions easily conceived within the technical scope disclosed by the present invention shall fall within the protection scope of the present invention.

Industrial Applicability

It can be used to avoid serious current imbalance between the first branch and the second branch connected in parallel in the high-voltage discharge circuit, thereby improving system stability and equipment safety. In other words, the current detection device proposed in the present invention is provided with a processor which can monitor and handle the over-current issue during discharge in the first and second branches. At the same time, by presetting thresholds through the first, second and third digital-to-analog converters (bias voltage signal, first reference comparison voltage, second reference comparison voltage), there is no need to concern oneself with the discharge process; one only needs to read the electrical interface level state of the processor to make a judgment. The presetting of the thresholds allows system parameter errors to exist during product production and testing, giving high flexibility. In addition, the current detection module can be equipped with PCB Rogowski coils, which are electrically isolated from the measured conductors (first and second branches), do not consume primary-side energy during measurement, and do not alter the original electrical circuit due to non-contact measurement. Moreover, the absence of a core structure avoids magnetic saturation issues, and PCB Rogowski coils feature high manufacturing precision, good consistency, low production cost, and do not need to be connected to the high-voltage discharge circuit, avoiding the impact of the high-voltage circuit on the measured circuit, thereby ensuring high safety.

**Claims**

1. A current detection device, **characterized in that** the current detection device comprises:

a current detection module (110), arranged in a high-voltage discharge circuit, the current detection module (110) configured to perform current detection on a first branch and a second branch of the high-voltage discharge circuit so as to respectively obtain a first current signal ($I_2$) and a second current signal ($I_3$);
a processing module (130), coupled to the current detection module (110), when a current difference between the first current signal ($I_2$) and the second current signal ($I_3$) is not within an allowable range, the processing module (130) generating a discharge anomaly prompt signal;
a control module (140), connected to the processing module (130) and the current detection module (110), configured to send a pulse signal so as to disconnect the first branch and the second branch, upon receiving the discharge anomaly prompt signal sent by the processing module (130).

2. The current detection device according to claim **1, characterized in that** the current detection device comprises: a differential amplification circuit (120), connected between the current detection module (110) and the processing module (130), configured to perform differential amplification on the first current signal ($I_2$) and the second current signal ($I_3$).

3. The current detection device according to claim **1, characterized in that** the current detection module (110) comprises:

a first PCB Rogowski coil ($L_1$), the first PCB Rogowski coil ($L_1$) generating a first coil output voltage ($U_1$) based on the first current signal ($I_2$);
a second PCB Rogowski coil ($L_2$), the second PCB Rogowski coil ($L_2$) generating a second coil output voltage ($U_2$) based on the second current signal ($I_3$);
wherein, based on a difference between the first coil output voltage ($U_1$) and the second coil output voltage ($U_2$), the current difference between the first current signal ($I_2$) and the second current signal ($I_3$) is judged as to whether it is within the allowable range.

4. The current detection device according to claim 3, **characterized in that** the current detection device (100) further comprises:

a measured resistor (RS), a first terminal of the measured resistor (RS) connected to a first terminal of the first PCB Rogowski coil ($L_1$), a second terminal of the measured resistor (RS) connected to a first terminal of the second PCB Rogowski coil ($L_2$), and a potential difference (Uba) existing across the fist terminal and the second terminal of the measured resistor (RS); wherein, a second terminal of the first PCB Rogowski coil ($L_1$) is connected to a second terminal of the second PCB Rogowski coil ($L_2$), and winding directions of the first PCB Rogowski coil ($L_1$) and the second PCB Rogowski coil ($L_2$) are opposite, so that directions of the first coil output voltage ($U_1$) and the second coil output voltage ($U_2$) are opposite;
the control module (140) respectively transmits the pulse signal to a control terminal of a second switching element ($Q_2$) and a control terminal of a first switching element ($Q_1$) via a fourth resistor ($R_4$) and a fifth resistor (Rs); wherein, the first switching element ($Q_1$) is arranged in the first branch, a first terminal of the first switching element ($Q_1$) receives the first current signal ($I_2$) via a first current-sharing resistor ($R_1$), the second switching element ($Q_2$) is arranged in the second branch, a first terminal of the second switching element ($Q_2$) receives the second current signal ($I_3$) via a second current-sharing resistor ($R_2$), second terminals of the first switching element ($Q_1$) and the second switching element ($Q_2$) are both connected to a third resistor ($R_3$);
wherein, when the current difference between the first current signal ($I_2$) and the second current signal ($I_3$) is not

within the allowable range, the pulse signal sent by the control module (140) is used to disconnect the first switching element ($Q_1$) and the second switching element ($Q_2$);

when the current difference between the first current signal ($I_2$) and the second current signal ($I_3$) is within the allowable range, the pulse signal sent by the control module (140) after reaching a preset duration is used to disconnect the first switching element ($Q_1$) and the second switching element ($Q_2$);

when a current value of the first current signal ($I_2$) is equal to a current value of the second current signal ($I_3$), magnitudes of the first coil output voltage ($U_1$) and the second coil output voltage ($U_2$) are the same, and the current flowing through the measured resistor (RS) is zero;

when the current value of the first current signal ($I_2$) is greater than the current value of the second current signal ($I_3$), the first coil output voltage ($U_1$) is greater than the second coil output voltage ($U_2$), a first induced current (IS1) is greater than a second induced current (IS2), the current flowing through the measured resistor (RS) is the first induced current (IS1) minus the second induced current (IS2), and a first terminal potential (Ua) of the measured resistor (RS) is higher than a second terminal potential (Ub);

when the current value of the first current signal ($I_2$) is less than the current value of the second current signal ($I_3$), the first coil output voltage ($U_1$) is less than the second coil output voltage ($U_2$), the first induced current (IS1) is less than the second induced current (IS2), the current flowing through the measured resistor (RS) is the second induced current (IS2) minus the first induced current (IS1), and the first terminal potential of the measured resistor (RS) (Ua) is lower than the second terminal potential (Ub).

**5.** The current detection device according to claim **4, characterized in that**, the current detection device (100) comprises a differential amplification circuit (120), the differential amplification circuit (120) comprising:

a first comparator ($A_1$), a first input terminal of the first comparator ($A_1$) connected to the first terminal of the measured resistor (RS), a second input terminal of the first comparator ($A_1$) connected to a first terminal of a sixth resistor (RG);

a second comparator ($A_2$), a first input terminal of the second comparator ($A_2$) connected to a second terminal of the sixth resistor (RG), a second input terminal of the second comparator ($A_2$) connected to the second terminal of the measured resistor (RS);

a first differential resistor (RF1), a first terminal of the first differential resistor (RF1) connected to the first terminal of the sixth resistor (RG) and the second input terminal of the first comparator ($A_1$), a second terminal of the first differential resistor (RF1) connected to an output terminal of the first comparator ($A_1$);

a second differential resistor (RF2), a first terminal of the second differential resistor (RF2) connected to the second terminal of the sixth resistor (RG) and the first input terminal of the second comparator ($A_2$), a second terminal of the second differential resistor (RF2) connected to an output terminal of the second comparator ($A_2$);

a third differential resistor (RF3), a first terminal of the third differential resistor (RF3) connected to the second terminal of the first differential resistor (RF1) and the output terminal of the first comparator ($A_1$);

a third comparator ($A_3$), a first input terminal of the third comparator ($A_3$) connected to a second terminal of the third differential resistor (RF3);

a fourth differential resistor (RF4), a first terminal of the fourth differential resistor (RF4) connected to the second terminal of the third differential resistor (RF3) and the first input terminal of the third comparator ($A_3$), a second terminal of the fourth differential resistor (RF4) connected to an output terminal of the third comparator ($A_3$);

a fifth differential resistor (RF5), a first terminal of the fifth differential resistor (RF5) connected to the second terminal of the second differential resistor (RF2) and the output terminal of the second comparator ($A_2$);

a sixth differential resistor (RF6), a first terminal of the sixth differential resistor (RF6) connected to a second terminal of the fifth differential resistor (RF5) and a second input terminal of the third comparator ($A_3$), a second terminal of the sixth differential resistor (RF6) receiving a bias voltage signal (Uref);

wherein, the first differential resistor (RF1) and the second differential resistor (RF2) are identical, and the third differential resistor (RF3), the fourth differential resistor (RF4), the fifth differential resistor (RF5) and the sixth differential resistor (RF6) are identical.

**6.** The current detection device according to claim 5, **characterized in that**, voltage difference (Udc) between output voltage (Uc) of the first comparator ($A_1$) and output voltage (Ud) of the second comparator ($A_2$) is expressed as:

$$U_{dc} = \frac{U_{ba}}{R_G} * (R_G + R_{F1} + R_{F2})$$

:

when the bias voltage signal (Uref) acts alone, first output voltage (Uout1) at the output terminal of the third comparator (A$_3$) is expressed as:

$$U_{out1} = U_{ref}$$

second input terminal voltage (Uf) of the third comparator (A$_3$) is divided voltage of the output voltage (Ud) of the second comparator (A$_2$) at the fifth differential resistor (RF5) and the sixth differential resistor (RF6); first input terminal voltage (Ue) of the third comparator (A$_3$) is divided voltage of second output voltage (Uout2) at the output terminal of the third comparator (A$_3$) at the third differential resistor (RF3) and the fourth differential resistor (RF4); when the output voltage (Ud) of the second comparator (A$_2$) acts alone,

$$\frac{U_d}{R_{F5} + R_{F6}} * R_{F6} = \frac{U_{out2}}{R_{F3} + R_{F4}} * R_{F3}$$

then the second output voltage (Uout2) at the output terminal of the third comparator (A$_3$) is expressed as:

$$U_{out2} = U_d * \frac{R_{F3} + R_{F4}}{R_{F5} + R_{F6}} * \frac{R_{F3}}{R_{F6}}$$

when the output voltage (Uc) of the first comparator (A$_1$) acts alone,

$$\frac{-U_c}{R_{F3} + R_{F4}} * R_{F4} = \frac{U_{out3}}{R_{F5} + R_{F6}} * R_{F6}$$

then the third output voltage (Uout3) at the output terminal of the third comparator (A$_3$) is expressed as:

$$U_{out3} = -U_c * \frac{R_{F5} + R_{F6}}{R_{F3} + R_{F4}} * \frac{R_{F4}}{R_{F6}}$$

output voltage (Uout) at the output terminal of the third comparator (A$_3$) is equal to the sum of the first output voltage (Uout1), the second output voltage (Uout2) and the third output voltage (Uout3) at the output terminal of the third comparator (A$_3$), and is expressed as:

$$U_{out} = U_{out1} + U_{out2} + U_{out3} = U_{ref} + U_d * \frac{R_{F3} + R_{F4}}{R_{F5} + R_{F6}} * \frac{R_F}{R_F}$$

$$U_{out} = U_{ref} + U_d - U_c = U_{ref} + \frac{U_{ba}}{R_G} * (R_G + R_{F1} + I$$

wherein, amplification factor G is:

$$G = 1 + \frac{R_{F1} + R_{F2}}{R_G}$$

7. The current detection device according to claim **6, characterized in that**, the processing module (130) comprises:

a processor (MCU), connected to the control module (140); when the current difference between the first current ($I_2$) and the second current signal ($I_3$) is not within the allowable range, the processor (MCU) controls the pulse signal sent by the control module (140) to disconnect the first switching element ($Q_1$) and the second switching element ($Q_2$).

**8.** The current detection device according to claim **7, characterized in that** the processing module (130) further comprises:

a first digital-to-analog converter (DAC1), a first terminal of the first digital-to-analog converter (DAC1) connected to the processor, a second terminal of the first digital-to-analog converter (DAC1) connected to the second terminal of the sixth differential resistor (RF6) and providing the bias voltage signal (Uref);

a second digital-to-analog converter (DAC2), a first terminal of the second digital-to-analog converter (DAC2) connected to the processor (MCU);

a fourth comparator ($A_4$), a first input terminal of the fourth comparator ($A_4$) connected to a second terminal of the second digital-to-analog converter (DAC2) and receiving a first reference comparison voltage (Ucon1), a second input terminal of the fourth comparator ($A_4$) connected to the output terminal of the third comparator ($A_3$) and receiving the output voltage (Uout) at the output terminal of the third comparator ($A_3$);

a third digital-to-analog converter (DAC3), a first terminal of the third digital-to-analog converter (DAC3) connected to the processor (MCU);

a fifth comparator ($A_5$), a first input terminal of the fifth comparator ($A_5$) connected to the output terminal of the third comparator ($A_3$) and the second input terminal of the fourth comparator ($A_4$),

and receiving the output voltage (Uout) at the output terminal of the third comparator ($A_3$), a second input terminal of the fifth comparator ($A_5$) connected to a second terminal of the third digital-to-analog converter (DAC3) and receiving a second reference comparison voltage (Ucon2).. The current detection device according to claim **8, characterized in that** the processing module (130) further comprises:

a first diode ($D_1$), a first terminal of the first diode ($D_1$) connected to an output terminal of the fourth comparator ($A_4$);

a second diode ($D_2$), a first terminal of the second diode ($D_2$) connected to an output terminal of the fifth comparator ($A_5$);

a first bistable trigger ($QD_1$), a clock input terminal (CK) of the first bistable trigger ($QD_1$) connected to a second terminal of the first diode ($D_1$), a preset level signal terminal (PR), an input terminal (D), a clear signal terminal (CLR) and an output terminal (Q) of the first bistable trigger ($QD_1$) connected to the processor (MCU);

a second bistable trigger ($QD_2$), a clock input terminal (CK) of the second bistable trigger ($QD_2$) connected to a second terminal of the second diode ($D_2$), a preset level signal terminal (PR), an input terminal (D), a clear signal terminal (CLR) and an output terminal (Q) of the second bistable trigger ($QD_2$) connected to the processor (MCU);

wherein, when the current value of the first current signal ($I_2$) is greater than the current difference value of the second current signal ($I_3$), and the difference is not within the allowable range, the output voltage (Uout) at the output terminal of the third comparator ($A_3$) is lower than the first reference comparison voltage (Ucon1), the output terminal of the fourth comparator ($A_4$) is at a low level, the output terminal of the fifth comparator ($A_5$) is at a high level, the output terminal of the first bistable trigger ($QD_1$) is at a low level, the output terminal of the second bistable trigger ($QD_2$) is at a high level, and the processor (MCU) sends the discharge anomaly prompt signal to the control module (140) and disconnects the first switching element ($Q_1$) and the second switching element ($Q_2$);

wherein, when the current value of the first current signal ($I_2$) and the current difference value of the second current signal ($I_3$) are within the allowable range, the output voltage (Uout) at the output terminal of the third comparator ($A_3$) is between the first reference comparison voltage (Ucon1) and the second reference comparison voltage (Ucon2), the output terminal of the fourth comparator ($A_4$) is at a high level, the output terminal of the fifth comparator ($A_5$) is at a high level, the output terminal of the first bistable trigger ($QD_1$) is at a high level, the output terminal of the second bistable trigger ($QD_2$) is at a high level, and the processor (MCU) does not send the discharge anomaly prompt signal to the control module (140);

wherein, when the current value of the first current signal ($I_2$) is less than the current difference value of the second current signal ($I_3$) and the difference is not within the allowable range, the output voltage (Uout) at the output terminal of the third comparator ($A_3$) is higher than the second reference comparison voltage (Ucon2), the output terminal of the fourth comparator ($A_4$) is at a high level, the output terminal of the fifth comparator ($A_5$) is at a low level, the output terminal of the first bistable trigger ($QD_1$) is at a high level, the output terminal of the second bistable trigger ($QD_2$) is at a low level, and the processor (MCU) sends the discharge anomaly prompt signal to the control module (140) and disconnects the first switching element ($Q_1$) and the second switching element ($Q_2$).

10. A control method for a current detection device, **characterized in that**, the control method comprises:

performing current detection on a first branch and a second branch of a high-voltage discharge circuit so as to respectively obtain a first current signal and a second current signal;
when a current difference between the first current signal and the second current signal is not within an allowable range, generating a discharge anomaly prompt signal;
sending a pulse signal based on the discharge anomaly prompt signal so as to disconnect the first branch and the second branch.

11. A vascular calcification treatment apparatus, **characterized in that** it comprises the current detection device according to any one of claims **1** to **9,** and a solution tank; wherein two ends of the solution tank are respectively connected to positive and negative high voltages so as to form a high-voltage discharge circuit.

FIG. 1

FIG. 2

performing current detection on a first branch and a second branch of the high-voltage discharge circuit so as to respectively obtain a first current signal and a second current signal — 210

when the current difference between the first current signal and the second current signal is not within the allowable range, generating a discharge anomaly prompt signal — 220

sending a pulse signal based on the discharge anomaly prompt signal so as to disconnect the first branch and the second branch — 230

FIG. 3

start

MCU sets DAC to output reference comparison voltages to define the upper and lower limit voltages — S301

FPGA drives IGBT to close — S302

current flows the branches — S303

PCB coil generates coil output voltage — S304

coil output voltage differentially is amplified and biased — S305

output voltage is compared with the upper and lower limit voltages — S306

output the level states after comparing — S307

level states are stabilized by two bistable triggers — S308

S309

level states of the two bistable triggers are both high

YES

both high — S310

the current difference is within the allowable range — S311

FPGA controls the IGBTs to turn off after reaching the duration — S312

the high-voltage discharge is normal — S313

NO

S314 — QD1 is low?

YES

over-current event occurs in the first branch — S315

FPGA immediately controls the IGBTs to turn off — S316

MCU generates a discharge anomaly prompt signal — S317

NO

over-current event occurs in the second branch — S318

FPGA immediately controls the IGBTs to turn off — S319

MCU generates a discharge anomaly prompt signal — S320

end

FIG. 4

300

140

130

FPGA

MCU

HV+

310

solution tank

R1

R2

R4

pulse signal

Q1

Q2

R5

R3

HV-

FIG. 5

## INTERNATIONAL SEARCH REPORT

| International application No. |
|---|
| **PCT/CN2024/076190** |

**A.    CLASSIFICATION OF SUBJECT MATTER**

G01R19/00(2006.01)i;  A61B17/22(2006.01)i

According to International Patent Classification (IPC) or to both national classification and IPC

**B.    FIELDS SEARCHED**

Minimum documentation searched (classification system followed by classification symbols)

IPC:  G01R A61B

Documentation searched other than minimum documentation to the extent that such documents are included in the fields searched

Electronic data base consulted during the international search (name of data base and, where practicable, search terms used)

CNABS; CNTXT; CNKI; VEN; ENTXT; USTXT; WOTXT: 江苏朴瓩医疗科技, 唐智皇, 李闯, 周毅, 桂宝珠, 电流, 并联, 均流, 均压, 均衡, 差, 比较, 差分放大, 关, 断, 保护, 罗氏, 线圈, 绕, 反向, current, parallel, current sharing, voltage sharing, difference, compar+, differential amplification, off, protection, Rogowski, coil, winding, reverse

**C.    DOCUMENTS CONSIDERED TO BE RELEVANT**

| Category* | Citation of document, with indication, where appropriate, of the relevant passages | Relevant to claim No. |
|---|---|---|
| PX | CN 115993477 A (SHANGHAI PAF MEDTECH CO., LTD.) 21 April 2023 (2023-04-21) claims 1-11 | 1-11 |
| X | CN 114362487 A (XIDIAN UNIVERSITY) 15 April 2022 (2022-04-15) description, paragraphs [0001]-[0060], and figures 1-6 | 1-3, 10, 11 |
| X | CN 107565802 A (BEIJING RESEARCH INSTITUTE OF PRECISE MECHATRONICS AND CONTROLS) 09 January 2018 (2018-01-09) description, paragraphs [0001]-[0043], and figures 1-3 | 1-3, 10, 11 |
| A | JP 2004229382 A (TOSHIBA CORP.) 12 August 2004 (2004-08-12) entire document | 1-11 |
| A | CN 206892706 U (HARBIN UNIVERSITY OF SCIENCE AND TECHNOLOGY) 16 January 2018 (2018-01-16) entire document | 1-11 |
| A | CN 114552718 A (FUJIAN CONTEMPORARY NEBULA ENERGY TECHNOLOGY CO., LTD.) 27 May 2022 (2022-05-27) entire document | 1-11 |

☐ Further documents are listed in the continuation of Box C.     ☑ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | "&" | document member of the same patent family |
| "P" | document published prior to the international filing date but later than the priority date claimed | | |

| Date of the actual completion of the international search | Date of mailing of the international search report |
|---|---|
| **08 April 2024** | **20 April 2024** |

| Name and mailing address of the ISA/CN | Authorized officer |
|---|---|
| **China National Intellectual Property Administration (ISA/CN)** **China No. 6, Xitucheng Road, Jimenqiao, Haidian District, Beijing 100088** | |
| | Telephone No. |

Form PCT/ISA/210 (second sheet) (July 2022)

## INTERNATIONAL SEARCH REPORT
### Information on patent family members

International application No.

**PCT/CN2024/076190**

| Patent document cited in search report | | | Publication date (day/month/year) | Patent family member(s) | Publication date (day/month/year) |
|---|---|---|---|---|---|
| CN | 115993477 | A | 21 April 2023 | None | |
| CN | 114362487 | A | 15 April 2022 | None | |
| CN | 107565802 | A | 09 January 2018 | None | |
| JP | 2004229382 | A | 12 August 2004 | None | |
| CN | 206892706 | U | 16 January 2018 | None | |
| CN | 114552718 | A | 27 May 2022 | None | |

Form PCT/ISA/210 (patent family annex) (July 2022)

**EP 4 667 947 A1**

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 202310126146X